(19) Europäisches Patentamt
European Patent Office
Office européen des brevets

(11) EP 0 535 387 B2

(12) NEUE EUROPÄISCHE PATENTSCHRIFT

(45) Veröffentlichungstag und Bekanntmachung des Hinweises auf die Entscheidung über den Einspruch:
**04.10.2001 Patentblatt 2001/40**

(45) Hinweis auf die Patenterteilung:
**21.08.1996 Patentblatt 1996/34**

(21) Anmeldenummer: **92114981.1**

(22) Anmeldetag: **02.09.1992**

(51) Int Cl.[7]: **A61K 49/00**

# (54) Echogene Partikel, Verfahren zu deren Herstellung und deren Verwendung

Echographique particle, preparation and application

Particule échographique, préparation et application

(84) Benannte Vertragsstaaten:
**AT BE CH DE DK ES FR GB GR IE IT LI LU MC NL PT SE**

(30) Priorität: **03.09.1991 DE 4129187**
**24.03.1992 DE 4209487**
**24.03.1992 DE 4209488**

(43) Veröffentlichungstag der Anmeldung:
**07.04.1993 Patentblatt 1993/14**

(73) Patentinhaber: **HOECHST AKTIENGESELLSCHAFT**
**65926 Frankfurt am Main (DE)**

(72) Erfinder:
- **Krone, Volker, Dr.**
  **W-6238 Hofheim (Taunus (DE)**
- **Walch, Axel, Dr.**
  **W-6000 Frankfurt am Main 90 (DE)**
- **Grande, Jürgen**
  **W-6237 Liederbach (DE)**
- **Zotz, Rainer, Dr.**
  **W-6200 Wiesbaden (DE)**

(74) Vertreter: **Zinngrebe, Horst, Dr.rer.nat.**
**Saalbaustrasse 11**
**64283 Darmstadt (DE)**

(56) Entgegenhaltungen:

| | |
|---|---|
| **EP-A- 0 324 938** | **EP-A- 0 458 079** |
| **EP-A- 0 458 745** | **WO-A-88/07870** |
| **WO-A-89/06978** | **WO-A-91/16080** |
| **WO-A-92/08496** | **DE-A- 4 219 723** |
| **GB-A- 1 516 348** | **US-A- 4 265 251** |
| **US-A- 4 718 433** | **US-A- 4 900 540** |
| **US-A- 4 957 656** | |

- **K. MAKINO et al.; Chem. Pharm. Bull., vol. 33, (1985), S. 1195-1201**
- **EMBLETON et al.; J. Microencapsulation, vol. 9(1), (1992), pp. 73-87**
- **C. CHRISTIANSEN et al.; Biotechnol. Appl. Biochem., vol. 19, (1994), pp. 307- 312**
- **DARNELL et al.; Molecular Cell Biology, Second Edition, pp. 510-512**
- **N. MOHANDAS et al.; Seminars in Hematology, vol. 20(3), (1983), pp. 225-228**
- **McGRAW-HILL DICTIONARY OF SCIENTIFIC AND TECHNICAL TERMS, Fifth Edition, (1993), p. 1127**
- **J. BARNHART et al.; Investigative Radiology, vol. 25, Sept. 1990, pp. S162-164**
- **Philip M. MORSE: "Theoretical Acoustics"; Princeton University Press (1986)**
- **P.N.T. WELLS: "Physical Principles of Ultrasonic Diagnosis"; Academic Press (1969)**
- **M. J. MONAGHAN: "Practical Echocardiography and Doppler"; John Wiley & Sons (1990)**

Bemerkungen:
Die Akte enthält technische Angaben, die nach dem Eingang der Anmeldung eingereicht wurden und die nicht in dieser Patentschrift enthalten sind.

EP 0 535 387 B2

## Beschreibung

Die Erfindung betrifft echogene Partikel, enthaltend ein Gas und eine formgebende Substanz, wobei die Oberfläche der Partikel im wesentlichen glatt ist und die Form keine oder 1 bis 10 konkave Oberflächensegmente enthält oder die Oberfläche nicht glatt ist und die Form 1 bis 10 konkave Oberflächensegmente enthält und die Lunge im Blutkreislauf passieren können, ein Sprühverfahren zu deren Herstellung und deren Verwendung insbesondere als Diagnostika und Therapeutika.

Die Ultraschalldiagnostik hat in der Medizin wegen der komplikationslosen einfachen Handhabung sehr breite Anwendung gefunden. Ultraschallwellen werden an Grenzflächen von unterschiedlichen Gewebearten reflektiert. Die dabei entstehenden Echosignale werden elektronisch verstärkt und sichtbar gemacht.

Die Darstellung von Blutgefäßen und inneren Organen mittels Ultraschall erlaubt im allgemeinen nicht die Darstellung des darin vorhandenen Blutflusses. Flüssigkeiten, insbesondere Blut, liefern nur dann Ultraschalikontrast, wenn Dichteunterschiede zur Umgebung bestehen. Als Kontrastmittel werden in der medizinischen Ultraschalldiagnostik z.B. Gase enthaltende oder Gase produzierende Substanzen verwendet, da der Impedanzunterschied zwischen Gas und umgebendem Blut wesentlich größer ist, als der zwischen Flüssigkeiten oder Festkörpern und Blut (Levine R.A., J. Am. Coll. Cardiol 3:28, 1989; Machi, i.J. CU 11:3, 1983).

In der Literatur sind mehrere Methoden zur Herstellung und Stabilisierung von Gasblasen bekannt. In dem US-Patent 4,276,885 wird die Herstellung von Gasbläschen mit definierter Größe beschrieben, die mit einer vor dem Zusammenfließen der Gasblasen schützenden Gelatinehülle umgeben sind. Die Aufbewahrung der fertigen Gasbläschen kann nur im eingefrorenen Zustand erfolgen, wobei die Gasbläschen zur Verwendung wieder auf Körpertemperatur gebracht werden müssen.

Die EP-A2-0123 235 und 0 122 624 beschreiben Gase enthaltende Ultraschall-Kontrastmittel, die aus Mischungen von grenzflächenaktiven Substanzen mit einem Feststoff in einem flüssigen Träger bestehen. Die Ultraschall-Kontrastmittel werden durch einen aufwendigen Vermahlungsprozeß mit einer Luftstrahlmühle hergestellt. Die so hergestellten Partikel haben nur eine geringe Nutzungsdauer, weil sie die eingeschlossenen Gase schnell verlieren.

Die EP-A2-0 224 934 beschreibt Ultraschall-Kontrastmittel in Form von gasgefüllten Gelatine- oder Albuminhohlkörpern. Nachteilig ist jedoch die Verwendung von körperfremden oder denaturierten körpereigenen Proteinen wegen des damit verbundenen allergenen Risikos.

Der vorliegenden Erfindung liegt die Aufgabe zugrunde, echogene Partikel zu finden, die Ultraschallwellen reflektieren, absorbieren, beugen bzw. streuen und so einen deutlichen Kontrast zum umgebenden Gewebe liefern, die so klein und stabil sind, daß sie ohne wesentlichen Gasverlust und im wesentlichen quantitativ die linke Herzkammer nach intravenöser Applikation erreichen und sich schnell und einfach herstellen lassen.

Es wurde gefunden, daß die Qualität von echogenen Partikeln besonders von der Form und der Oberfläche dieser Partikel abhängt. Es zeigt sich, daß die echogenen Partikel mit konkaven Oberflächensegmenten die Lunge im Blutkreislauf wesentlich besser passieren können und einen höheren Kontrast im Ultraschall bewirken. Diese echogenen Partikel behalten eine Luft-Wasser-Grenzfläche, deren Streuquerschnitt bei Ultraschalluntersuchungen sehr hoch ist. Echogene Partikel mit im wesentlichen glatter Oberfläche können die Lungen von Wirbeltieren im Blutkreislauf passieren ohne daß die Partikel im großen Maße ausfiltriert werden.

Die Erfindung betrifft somit echogene Partikel, enthatend ein Gas und eine formgebende Substanz oder ein Substanzengemisch, die/das biologisch abbaubar und/oder ausscheidbar ist, und im Blutkreislauf die Lunge von Wirbeltieren passieren können, dadurch gekennzeichnet, daß (a) die Oberfläche des echogenen Partikels im wesentlichen glatt ist und die Form des echogenen Partikels keine oder 1 bis 10 konkave Oberflächensegmente enthält oder die Oberlfäche des echogenen Partikels nicht glatt ist und die Form des echogenen Partikels 1 bis 10 konkave Oberflächensegemente enthält, und daß (b) die maximale Einwölbung des konkaven Oberflächensegments von 30 % bis 90 % des kleinsten Partikelquerschitts beträgt und 10 bis 60 % der echogenen Partikel mindestens ein konkaves Oberflächensegment pro Partikel aufweisen.

Echogene Partikel sind Partikel mit einem Durchmesser, der bei 90 % der Partikel von 0,1 µm bis 20 µm reicht, bevorzugt von 0,2 µm bis 7 µm, insbesondere von 0,5 µm bis 3 µm. Diese Partikel sind ferner in der Lage, Schallwellen zu reflektieren, absorbieren, beugen bzw. zu streuen.

Mit konkaven Oberflächensegment eines echogenen Partikels ist ein Partikel gemeint, das mindestens eine nach innen gewölbte Oberfläche aufweist. Zu den erfindungsgemäßen Partikeln gehören beispielsweise Partikel, bei denen die maximale Einwölbung d.h. die Tiefe des konkaven Oberflächensegments von 30 % bis 90 % des kleinsten Querschnitts des Partikels beträgt, besonders bevorzugt sind Einwölbungen von 50 bis 90 %. Es sind also im wesentlichen Partikel gemeint, die becher-, hut- oder topfförmig aussehen.

Die Anzahl der konkaven Oberflächensegmente reicht von 1 bis 10, bevorzugt 1 bis 5, insbesondere 1 bis 3.

Die Partikel können auch eine glatte Oberfläche aufweisen. Unter einer im wesentlichen glatten Oberfläche von einem echogenen Partikel sind Oberflächen gemeint, die bei einer 10 000-fachen Vergrößerung keinerlei Strukturunterschiede auf der Oberfläche erkennen lassen, d.h. Störungen auf der Oberfläche dieser Partikel wie Erhebungen

oder Vertiefungen haben im wesentlichen weniger als 100 nm Abstand von der mittleren Oberfläche des Partikels, insbesondere 90 bis 50 nm, bevorzugt 60 bis 80 nm. Die mittlere Partikeloberfläche ergibt sich aus dem Durchschnitt der Erhebungen und Vertiefungen der Oberfläche. Zu den Störungen der Oberfläche gehören Veränderungen der homogenen Oberfläche, die im elektronenmikroskopischen Bild aussehen wie Erhebungen, Brüche, Verwerfungen, treppen, stufen-, schuppen- oder schichtförmiger Aufbau der Oberfläche sowie Löcher, Vertiefungen oder Poren in der Oberfläche.

Die Eigenschaft der erfindungsgemäßen Partikel, die Lunge von Wirbeltieren im Blutkreislauf passieren zu können, wird beispielsweise festgestellt, indem die echogenen Partikel unter sterilen Bedingungen in eine periphere Vene eines Versuchstiers injiziert werden. Ein Ultraschallkopf eines Ultraschallgerätes wird an den Thorax des Versuchstieres gehalten, so daß ein typischer Querschnitt durch die rechte und linke Herzkammer erhalten wird. Sobald das Ultraschall-Kontrastmittel die rechte Herzkammer erreicht, kann man auf dem Monitor des Ultraschallgerätes erkennen, wie das durch das Kontrastmittel markierte Blut den rechten Vorhof erreicht, dann den rechten Ventrikel und anschließend das Herz über die Pulmonalarterie wieder verläßt. Nach der Lungenpassage wird in der linken Herzkammer ein signifikanter Kontrastanstieg durch das Kontrastmittel erkennbar.

Die echogenen Partikel enthalten Gas beispielsweise Luft, Stickstoff, Edelgas wie Helium, Neon, Argon oder Krypton, Wasserstoff, Kohlendioxid, Sauerstoff, oder deren Gemische. Die echogenen Partikel werden mit einem Gas beladen, indem beispielsweise die Partikel in einer entsprechenden Gasatmosphäre gelagert oder bei der Sprühtrokkung direkt bei der Herstellung in einer entsprechenden Gasatmosphäre gewonnen werden.

Unter formgebenden Substanzen oder Substanzengemischen werden alle Verbindungen verstanden, mit denen man Partikel herstellen kann. Dazu gehören beispielsweise natürlich vorkommende Polymere oder synthetisch herstellbare Polymere, Lipide, die in wäßrigen Lösungen Mizellen bilden, oder niedermolekulare Aminosäuren, Fette, oder Kohlenhydrate.

Unter biologisch abbaubar und/oder ausscheidbarer formgebender Substanz oder Substanzengemisch werden Substanzen verstanden, die im Körper von Tieren Menschen oder Pflanzen zu untoxischen Abbauprodukten umgewandelt werden oder ausgeschieden werden. Der Abbau kann enzymatisch, hydrolytisch oder durch teilweise oder vollständiges Lösen der Substanzen oder Zerfall der Partikel in kleinere Untereinheiten im Wasser erfolgen. Die Ausscheidung der Abbauprodukte erfolgt über Niere, Haut, Lunge oder Darm. Wenn die Substanzen ganz oder teilweise in Wasser in Lösung gehen oder nicht abgebaut werden, beispelsweise bei niedermolekularen Kohlenhydraten erfolgt die Ausscheidung ohne Abbau über die Niere.

Formgebende Substanzen oder Substanzgemische, die biologisch abbaubar und/oder ausscheidbar sind, werden aus der folgenden Gruppe ausgewählt:

Polyester,
von α-, β-, γ- oder ε-Hydroxycarbonsäure, beispielsweise Poly-ε-caprolacton, Polyhydroxybuttersäure, Polymilchsäure oder Polyhydroxyvaleriansäure,
Copolymere
der obengenannten Polyester untereinander oder mit Polyglykolsäure,
Polykondensate
die 2,3-O-Alkylidenweinsäure, 2,3-O-Alkyliden-L-threitol, Furo-[2,5]-gruppen oder Terephthalate enthalten, Polyaminodicarbonsäure-co-imide
wie Polysuccinimid-co-α,β-(butyloxycarbonylpropionyloxyethyl)-D,L-aspartamid,
Polyaminosäuren
wie Polyglutaminsäure, Polyasparaginsäure oder Polylysin, die basisch oder sauer in wäßriger Lösung reagieren, sowie deren Derivate,
Polyamide,
beispielsweise Polylysinmethylesterfumarsäure-co-glutaramid oder Polyhydroxyethylaspartamid,
Polyorthoester, Polyanhydride,
z. B. solche, die aus Sebacinsäure und Bis(p-carboxyphenoxy)propan zusammengesetzt sind,
Gelatine,
z. B. hydrophob modifizierte,
Albumine,
z. B. Rinderserumalbumin,
Polysaccharide,
z. B. Dextrane, Stärke oder basische oder saure Derivate davon,
Polymethylenmalonat, Zucker,
z. B. Glucose, Galaktose, Laktose oder Saccharose,
Fettsäuren
mit 8 bis 20 Kohlenstoffatomen,

Aminosäuren
wie proteinogene Aminosäuren und deren hydrophobe Derivate,
Alkohole
mit 12 bis 20 Kohlenstoffatomen oder
Lipide
wie Phospholipide.

Besonders bevorzugt enthalten die erfindungsgemäßen echogenen Partikel biologisch abbaubare und/oder ausscheidbare Polymere aus folgender Gruppe:
Polyamide, Polykondensate, Polyhydroxybuttersäure oder Polyaminodicarbonsäure-co-imide.
Die obengenannten formgebenden Substanzen oder Substanzengemische lassen sich nach literaturbekannten Verfahren herstellen (EP-0,327,490; EP-0,398,935; EP-0,458,079; EP-0,123,235; EP-0,224,934; EP-0,245,840, M. Bornschein et al.,Die Pharmazie, 49, Heft 9, 1989, S.585ff.; J.W. Freytag, J. Microencapsulation, 2, Heft 1,1985, S.31-38; Y Kawashima et al., J. Pharmaceutical Sciences, 81, Heft 2, 1992, S. 135 ff., EP 0,077,752; EP 0,131,540).
Die obengenannten Polykondensate enthalten im wesentlichen mindestens 95 Mol-% an wiederkehrenden Struktureinheiten der Formel I

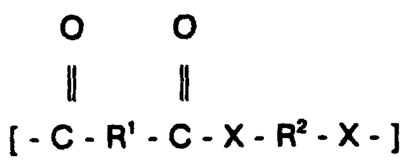

(I)

wobei $R^1$ für

a) Verbindung der Formel II

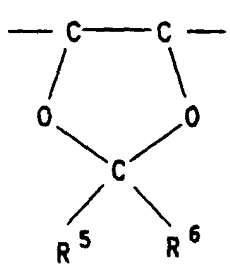

(II)

wobei $R^5$ und $R^6$ inerte Reste bedeuten,
b) geradkettiges oder verzweigtes Alkyl, Alkenyl, Cycloalkyl oder Cycloalkenyl, die mit einem oder mehreren inerten Resten substituiert sein können,
c) Aryl, ein- oder mehrkernig, ein- oder mehrfach substituiert durch inerte Reste,
d) Verbindungen der Formel V,

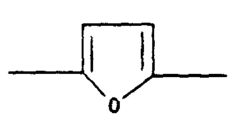

(V)

e) heterocyclischer Rest, der ein- oder mehrkernig und ein- oder mehrfach durch inerte Reste substituiert sein kann, steht

wobei X für

a) - O-,
b) - NH - oder
c)-S-

steht,
wobei $R^2$ für

a) Verbindung der Formel III,

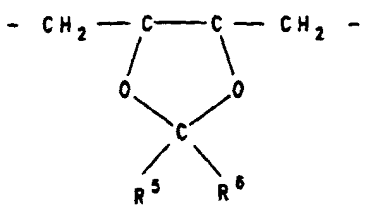

(III)

worin $R^5$ und $R^6$ die obengenannte Bedeutung haben,
b) geradkettiges oder verzweigtes Alkyl, Alkenyl, Cycloalkyl oder Cycloalkenyl, die mit einem oder mehreren inerten Resten substituiert sein können,
c) ein- oder mehrkerniges Aryl, ein- oder mehrfach substituiert durch inerte Reste,
d) heterocyclischer Rest, der ein- oder mehrkernig und ein- oder mehrfach durch inerte Reste substituiert sein kann, oder
e) Verbindung der Formel VI,

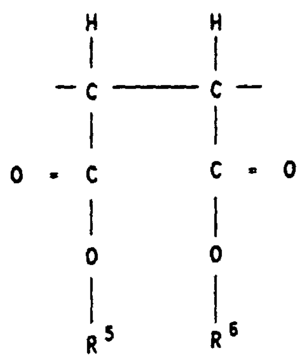

(VI)

worin $R^5$ und $R^6$ die obengenannte Bedeutung haben,
mit der Maßgabe, daß mehr als 5 Mol-% der Reste $R^1$ und/oder $R^2$ in dem Polykondensat der Formel I enthalten sind, wobei $R^1$ für eine Verbindung der Formel II und/oder $R^2$ für eine Verbindung der Formel III steht, oder daß mehr als 5 Mol-% der Reste $R^1$ und $R^2$ in dem Polykondensat der Formel I enthalten sind, wobei

a) $R^1$ für eine Verbindung der Formel V und $R^2$ für eine Verbindung der Formel VI steht, oder
b) $R^1$ für Phenyl[1,2], Phenyl[1,3] oder Phenyl[1,4] und $R^2$ für eine Verbindung der Formel VI steht.

Unter dem Begriff "inerte Reste" werden Substituenten verstanden, die unter den Herstellungs- und Verarbeitungsbedingungen der erfindungsgemäßen Polykondensate nicht miteinander reagieren oder durch Schutzgruppen daran gehindert werden miteinander zu reagieren. Inerte Reste können beispielsweise anorganische Reste wie Halogen sein, oder es kann sich um organische Reste wie Alkyl, Alkenyl, Cycloalkyl, Cycloalkenyl, Alkoxy, oder Dialkylaminoalkyl handeln.
Funktionelle Gruppen, die durch Schutzgruppen an der Reaktion gehindert werden, sind beispielsweise Amino oder Hydroxy.
Bekannte Schutzgruppen sind z. B. die Benzyloxy- oder Phenylsulfonylgruppe.
Heterocyclische Reste können ein- oder mehrkernig sein und weisen insbesondere ein oder zwei Sauerstoff-,
**[0027]** Stickstoff- oder Schwefelatome im Kern auf.

Aryl steht für einen aromatischen Kohlenstoffring. Mehrkernige Aryl-Reste können miteinander kondensiert, über C-C-Bindungen oder über Brückengruppen wie z. B. -O-, -COO-, $-CH_2-$, -CO-NH-, -S-, -CO- oder eine $-SO_2-$Gruppe linear miteinander verbunden sein.

Beispiele für mehrkernige aromatische Aryl-Reste sind 4,4'-Biphenyl, Naphthalin-1,5- oder -2,6-Gruppen oder Naphthalin-1,8-Gruppen.

Zur Herstellung der obengenannten Polykondensate setzt man ein Dicarbonsäuredichlorid der Formel X,

$$ClOC - R^1 - COCl \qquad (X)$$

mit einem oder mehreren der Diole, Diamine, Dithioverbindungen oder Diazine der Formeln XI, XII, XIII oder XIV um,

$$HO - R^2 - OH \qquad (XI)$$

$$H_2N - R^2 - NH_2 \qquad (XII)$$

$$HS - R^2 - SH \text{ (XIII)} \qquad (XI)$$

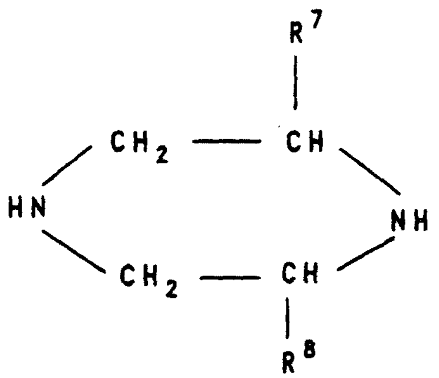

(XIV)

wobei $R^1$, $R^2$, $R^7$ und $R^8$ die obengenannte Bedeutung haben.

Das Dicarbonsäuredichlorid der Formel X und die einzelnen Diol-, Diamin-, Dithio- oder Diazintypen der Formeln XI, XII, XIII oder XIV können auch in Form von Mischungen miteinander eingesetzt werden.

Es ist für den Fachmann selbstverständlich, daß die Summe aller von den Dicarbonsäuren (A) abgeleiteten Struktureinheiten und die Summe aller von den Diolen, Dithioverbindungen, Diaminen und Diazinen (B) abgeleiteten Struktureinheiten im wesentlichen gleich sind, d. h., daß sie sich maximal um ca. 1 %, vorzugsweise maximal um 0,2 %, unterscheiden, insbesondere im Rahmen der praktischen Meß- und Dosierungsmöglichkeiten gleich sind.

Das Molekulargewicht der entstehenden Polyamide läßt sich unter anderem über die Auswahl der Mengenverhältnisse von A zu B steuern. Diese Auswahlkriterien sind dem Fachmann auf dem Gebiet der Polykondensation bekannt.

Beispiele für geeignete Dicarbonsäuren, von denen sich die Dicarbonsäuredichloride der Formel X ableiten, sind 2,3-O-Isopropyliden-L-weinsäure, 2-Chlorterephthalsäure, Furandicarbonsäure, 2-Bromterephthalsäure, 2-Methylterephthalsäure und insbesondere 2,3-O-Isopropyliden-L-weinsäure.

Beispiele für geeignete Diamine der Formel XII sind Lysinmethylester, Naphthalin-1,4-diamin, Naphthalin-1,5-diamin, Naphthalin-2,6-diamin und insbesondere Lysinmethylester oder Diazine.

Beispiele für geeignete Diole der Formel XI sind Weinsäurediethylester, 2,3-O-Isopropyliden-L-threitol und Weinsäurediisopropylester.

Die Kondensation der oben beschriebenen Komponenten wird im allgemeinen in Lösung ausgeführt.

Dazu werden die miteinander umzusetzenden monomeren Verbindungen in der Regel in einem organischen Lösungsmittel gelöst. Das organische Lösungsmittel enthält dabei vorzugsweise zumindest ein Lösungsmittel, wie z.

B. N-Methyl-2-pyrrolidon, N,N-Dimethylacetamid, Pyridin, Tetramethylharnstoff, N-Methyl-2-piperidon, Dichlormethan, N,N'-Dimethylethylenharnstoff, N-Methylcaprolactam, N-Acetylpyrrolidin, N,N-Dimethylpropylenharnstoff. Für das erfindungsgemäße Verfahren sind die bevorzugten organischen Lösungsmittel Pyridin, Dichlormethan, Furan oder eine Mischung dieser Verbindungen von Bedeutung.

Bei einer bevorzugten Form der Durchführung der Lösungspolymerisation werden die Monomeren 2,3-O-Isopropyliden-L-weinsäuredichloride und Weinsäurediethylester in einer Mischung von Pyridin und Dichlormethan unter heftigem Umrühren gemischt.

Die Polykondensationstemperaturen reichen bei der Lösungspolymerisation üblicherweise von - 20 °C bis + 120 °C, bevorzugt von + 10 °C bis + 100 °C. Besonders gute Ergebnisse werden bei Reaktionstemperaturen von + 10 °C bis + 80 °C erzielt.

Die Summe der Konzentrationen der monomeren Verbindungen in der Polymerisationsgemischlösung kann unter Beachtung des gewünschten Polymerisationsgrades, der gewünschten Viskosität des Polymerisationsgemisches, der Art der verwendeten monomeren Verbindungen, der Art des verwendeten Lösungsmittels und der gewünschten Polymerisationstemperatur eingestellt werden. Die günstigste Summe der Konzentrationen kann dabei aufgrund einer Reihe von Vorversuchen für den Ablauf der Polymerisation ermittelt werden.

Polykondensationsreaktionen werden vorzugsweise so ausgeführt, daß nach Abschluß der Reaktion 2 bis 15, vorzugsweise 5 bis 15 Gew.-% an Polykondensat in der Lösung vorliegen. Besonders gute Ergebnisse werden bei Konzentrationen von 5,0 bis 10 Gew.-% erzielt.

Im Verlauf der Polykondensation wächst das Molekulargewicht des Polymers und damit auch die Viskosität des Reaktionsansatzes an. Es werden Molekulargewichte von 500 bis 1.000.000 erreicht, bevorzugt 3.000 bis 100.000. Es werden in der Regel Viskositäten von mehr als 0,1 dl · $g^{-1}$ (Staudinger-Index, Dimethylformamid, 25 °C) erreicht.

Wenn die Polymerlösung die zur Weiterverarbeitung erforderliche Viskosität erreicht hat, kann die Polykondensation in üblicher Weise durch Zugabe von monofunktionellen Verbindungen, wie z. B. Acetylchlorid gestoppt werden. Anschließend kann der entstandene und locker an das Amidlösungsmittel gebundene Chlorwasserstoff durch Zugabe basischer Substanzen neutralisiert werden. Geeignet sind dafür beispielsweise Pyridin, Triethylamin, Morpholin, insbesondere aber Pyridin.

Die als Ausgangssubstanz einsetzbaren 2,3-O-Alkyliden-L-weinsäuredichloride erhält man beispielsweise durch Umsetzung von Weinsäurealkylester mit 2,2-Dimethoxypropan zu den entsprechenden Weinsäuredialkylesteracetonketalen analog den von M. Carmack et al. (J. Org. Chem. 33, 1968, Seiten 2171 - 2173) beschriebenen Umsetzungen. Diese Weinsäuredialkyesteracetonketale werden mit Alkalihydroxiden in die entsprechenden Salze überführt und abschließend durch Umsetzung mit Phosphortrichlorid, Phosphorpentachlorid, Oxalylchlorid oder Thionylchlorid in die entsprechenden L-Weinsäuredichloridderivate überführt.

Die Herstellung von 2,3-O-Alkyliden-L-threitol erfolgt beispielsweise durch Umsetzung von Weinsäuredialkylesteracetonketal mit $LiAlH_4$ zu dem entsprechenden 2,3-O-Alkyliden-2-threitol (P. W. Feit, J. Med. Chem. 7,1964, Seiten 14 - 17).

Furandicarbonsäuredichloride sind beispielsweise nach dem von Moore and Kelley (American Chemical Society, 11, No. 3, 1978, Seite 568 - 573) beschriebenen Verfahren erhältlich.

Die Erfindung betrifft ferner ein Verfahren zur Herstellung der erfindungsgemäßen echogenen Partikel, das dadurch gekennzeichnet ist, daß die formgebende Substanz oder Substanzengemisch in einem geeigneten Lösungsmittel oder Lösungsmittelgemisch gelöst wird und die Lösung einer Sprühtrocknung unterworfen wird.

Ferner können auch andere Verfahren, z. B. Emulsionsverfahren oder Fällungsverfahren zur Herstellung der erfindungsgemäßen Partikel eingesetzt werden (M. Bornschein et al, Die Pharmazie, 44, Heft 9, 1989, S. 585 ff.; Y-Kawashima et al., J. Pharm. Sciences, 81, Heft 2, 1992, S. 135 ff.)

Die Sprühtrocknung erfolgt beispielsweise nach dem Prinzip der Düsenversprühung im Gleichstrom, d.h. die Richtung der zu versprühenden Lösung und die Trockenluft verlaufen in der gleichen Richtung.

Zur Herstellung der erfindungsgemäßen echogenen Partikel haben sich folgende Einstellungen der wesentlichen Einstellparameter an einem Sprühtrockner (Büchi 190; Büchi GmbH, Eislingen), als geeignet erwiesen. Die Eingangstemperatur beträgt 15 °C bis 200 °C, insbesondere 20°C bis 100°C. Die Temperatur kann aber auch in weiten Grenzen schwanken und hängt im wesentlichen vom verwendeten Lösungsmittel und Polymer ab.

Unter Eingangstemperatur ist die Temperatur der Trocknungsluft, welche mit Hilfe des Aspirators durch das Gerät gesaugt wird, zu verstehen. Anstelle von Luft kann natürlich auch ein anderes Gas, sofern die durchzuführende Arbeit dies verlangt, zum Einsatz gelangen.

Beim Sprühtrocknen einer Lösung wird das Lösungsmittel ganz oder teilweise entfernt, dabei wandeln sich die flüssigen Lösungströpfchen in Partikel um. Damit nun das Lösungsmittel beim Versprühen des zu trocknenden Gutes in einen Luftstrom während der zur Verfügung stehenden Kontaktzeit verdampft, muß die Temperatur des Luftstromes über oder in der Nähe des Siedepunkts des Lösungsmittels liegen.

Als Ausgangstemperatur wird diejenige Temperatur bezeichnet, die der Luftstrom, welcher die Feststoffpartikel mit sich führt, vor dem Eintritt in den Zyklon aufweist. Die Ausgangstemperatur resultiert aus der Kombination

zwischen Eingangstemperatur, Aspiratoreinstellung, Pumpeneinstellung sowie Konzentration des Sprühgutes und ist nicht zuletzt auch abhängig von der Verdampfungswärme des Lösungsmittels.

Mit dem Aspirator wird die Trocknungsluft durch das Gerät hindurchgesaugt und dabei gleichzeitig ein Unterdruck erzeugt. Mit der Regulierung der Aspiratorleistung wird nun einerseits die Menge der aufgeheizten Trocknungsluft erhöht oder herabgesetzt und andererseits der in der Apparatur herrschende Unterdruck reguliert. Ein Druck, der im Bereich von 10 bis 100 mbar unterhalb des Drucks außerhalb der Apparatur ist, insbesondere von 30 bis 70 mbar, wird bevorzugt.

Die Aufgabe der Förderpumpe ist es, die Sprühlösung in das Gerät einzuspeisen, es haben sich Pumpenleistungen von 100 - 500 ml/h bewährt.

Als Sprühflow wird die zur Versprühung der Lösung, Emulsion oder Dispersion notwendige Menge Druckluft verstanden. Anstelle von Druckluft kann natürlich auch ein anderes Gas eingesetzt werden.

Der Sprühflow kann am Gerät im Bereich von 100 bis 800 Nl/h eingestellt werden (Nl = Normalliter) insbesondere 600 bis 800 Nl/h.

Als Lösungsmittel oder Lösungsmittelgemische eignen sich beispielsweise Wasser, Tetrahydrofuran, Dichlormethan, Furan, DMSO (Dimethylsulfoxid), Dioxan oder Aceton.

Zeigt das Sprühprodukt nach einer Probesprühung eine nicht glatte Oberfläche, so können durch den Zusatz von Polymerweichmachern wie DMSO oder Glycerin zum Lösungsmittel glatte Oberflächen hergestellt werden.

Zeigt das Sprühprodukt nach einer Probesprühung glatte Oberflächen und runde Partikel, so können durch Zusatz von Fällungsmitteln und/oder Temperaturveränderungen konkave Oberflächeneinwölbungen erzeugt werden.

Die Form der erfindungsgemäßen Partikel ergibt sich im wesentlichen aus dem Herstellverfahren und der Wahl der Parameter. Die erfindungsgemäßen Partikel mit konkaven Oberflächensegmenten leiten sich beispielsweise von runden Partikeln mit einem konstanten Durchmesser oder Hohlkörpern ab. Es kommen auch Formen vor, die wie Becher, Hüte, Zigarren oder Zylinder aussehen. Die Partikel können monolitisch sein oder als Hohlkörper vorliegen, z.B. als hohle eingewölbte Halbkugeln. Monolitische Partikel sind solche, die im wesentlichen von der formgebenden Substanz angefüllt sind; Hohlkörper haben einen mehr oder weniger großen gasgefüllten Innenraum. Hohle eingewölbte halbkugelförmige Partikel können durch mehr oder minder starke Einstülpungen der Hohlkugeln entstehen. Ferner haben sich spindelförmige Formen mit zwei Einstülpungen eines runden Partikels als günstig erwiesen. Alle genannten Formen haben sich als geeignete echogene Partikel erwiesen.

Die Verteilung der Formen in einer durch Sprühtrocknung hergestellten Charge kann in weiten Grenzen schwanken. Als günstig haben sich Chargen erwiesen, in denen insbesondere 30 bis 40 % der Partikel, mindestens ein konkaves Oberflächensegment aufweisen. Besonders bevorzugt sind echogene Partikel, von denen mindestens 5 % nur ein konkaves Oberflächensegment aufweisen. Die Verteilung der Formen kann durch computergestützte Bildanalyse der rasterelektronischen Aufnahmen der Partikel bestimmt werden oder kann mit Hilfe eines Cytophotometers erfolgen.

Die erfindungsgemäßen echogenen Partikel können auch eine glatte Oberfläche aufweisen. Dieses Merkmal läßt sich beispielsweise durch elektronenmikroskopische Untersuchungen insbesondere durch Rasterelektronenmikroskopie (REM) bestimmen. Dazu werden die Partikel nach Standardmethoden für die Elektronenmikroskopie vorbereitet und kontrastiert. Als Kontrastmittel für REM-Aufnahmen haben sich Metallaufdampfungen z. B. mit Gold bewährt. Glatte Oberflächen von Partikeln erscheinen auf dem REM-Aufnahmen als homogene Flächen mit weitgehend konstantem Grauton. Störungen der Oberfläche erkennt man an unterschiedlichen Grautönen. Störungen haben einen Abstand von der mittleren Oberfläche der Partikel von mehr als 100 nm.

Die erfindungsgemäßen Partikel mit glatter Oberfläche sind nicht völlig frei von Störungen der Oberfläche. Die Anzahl der Störungen pro $\mu m^2$ reicht von 0 bis 4, bevorzugt 0,5 bis 3, insbesondere von 1 bis 3. Die Qualität der glatten Oberfläche läßt sich beispielsweise dadurch bestimmen, daß man auf den REM-Aufnahmen der erfindungsgemäßen Partikeln mit glatter Oberfläche quadratische Areale mit einer Kantenlänge von 0,5 µm auswählt und die Anzahl der Störungen der Oberfläche bestimmt. Eine glatte Oberfläche ist dadurch gekennzeichnet, daß bei einer zufälligen Auswahl von 10 quadratischen Arealen 0 bis 10 Störungen der Oberfläche festgestellt werden, bevorzugt im Mittel von 1 bis 7,5, insbesondere 2,5 bis 7,5.

Die Verteilung der erfindungsgemäßen Partikel mit glatter Oberfläche in einer durch Sprühtrocknung hergestellten Charge kann in weiten Grenzen schwanken. Als günstig haben sich Chargen erwiesen in denen mindestens 10 %, bevorzugt 10 bis 90 %, insbesondere 20 bis 60 %, insbesondere bevorzugt 30 bis 40 % der Partikel eine glatte Oberfläche aufweisen.

Bei dem beschriebenen Verfahren können die formgebenden Substanzen allein oder auch als Gemisch eingesetzt werden. Diese formgebenden Substanzen können auch in Mischung mit anderen biologisch abbaubaren und/oder biologisch verträglichen Polymeren, z.B. Dextrane, Polyethylenglykole, Hydroxyethylstärke und andere abbaubare oder ausscheidbare Polysaccharide oder physiologisch unbedenklichen Hilfsstoffen (z. B. DMSO, Glycerin oder andere Polymerweichmacher) eingesetzt werden.

Die Erfindung betrifft ferner Diagnostika und Therapeutika, die dadurch gekennzeichnet sind, daß sie die

erfindungsgemäßen echogenen Partikel, neben pharmazeutisch geeigneten und physiologisch verträglichen Trägerstoffen, Zusatz-und/oder Hilfsstoffen enthalten kann.

Die erfindungsgemäßen echogenen Partikel werden vor Applikation durch Zusatz von einem oder mehreren pharmazeutisch geeigneten und physiologisch annehmbaren Trägern und gegebenenfalls weiteren Zusatz- und/oder Hilfsstoffen in eine geeignete diagnostische oder therapeutische Darreichungsform überführt. Die echogenen Partikel werden beispielsweise vor Applikation durch Zusatz von Wasser und Mischen suspendiert.

Durch Zugabe osmotisch aktiver Substanzen, beispielsweise Kochsalz, Galaktose, Glukose, Fruktose, kann die physiologische Isotonie der Partikelsuspension hergestellt werden. Ferner können Suspensionshilfsstoffe wie Dextrane oder Zusatzstoffe wie kolloidale Infusionslösungen zur Plasmasubstitution den Partikelsuspensionen zugemischt werden.

Bei den beschriebenen Verfahren zur Herstellung der erfindungsgemäßen echogenen Partikel kann man Partikelgrößen erreichen, bei denen 90 % der Partikel einen Durchmesser von 0,1 µm bis 15 µm haben, insbesondere werden Partikelgrößenverteilungen erreicht werden, bei denen 90 % der Partikel kleiner als 3 µm sind. Größere Teilchen werden mit einem Siebgewebe, das eine Maschenweite von 1 µm bis 50 µm, insbesondere 3 µm bis 15 µm hat, ausgesiebt. Bei der Verwendung dieser echogenen Partikel als Ultraschall-Kontrastmittel zur Diagnose von Herz-Kreislauf-Erkrankungen haben sich Partikelgrößen von 0,1 µm bis 7 µm bewährt, vorteilhaft werden Partikelgrößen von 0,1 µm bis 3 µm eingesetzt. Die echogenen Partikel werden beispielsweise in die Blutbahn injiziert. Je Injektion werden 0,1 mg bis 1000 mg der echogenen Partikel, bevorzugt 1 mg bis 100 mg, eingesetzt.

Die echogenen Partikel können aber auch oral appliziert werden, um den Magen-Darm-Trakt einer Ultraschalluntersuchung zugänglich zu machen.

Die erfindungsgemäßen echogenen Partikel können sowohl für diagnostische als auch therapeutische Verfahren verwendet werden. Die Verwendung der erfindungsgemäßen echogenen Partikel ist nicht nur auf die Sichtbarmachung des Blutstroms im rechtsventrikulären Teil des Blutkreislaufs nach venöser Applikation beschränkt. Die echogenen Partikel können mit ausgezeichnetem Erfolg für die Untersuchung der linken Herzseite und des Myocards verwendet werden. Ferner ist es auch möglich, andere vom Blut versorgte Organe wie Leber, Milz, Niere oder Gehirn, mit diesen echogenen Partikeln sichtbar zu machen.

Die erfindungsgemäßen echogenen Partikel eignen sich aber auch zum Sichtbarmachen von Hohlräumen in Menschen, Tieren oder Pflanzen, beispielsweise Harnblase, Harnleiter, Gebärmutter, Vagina,Xylem oder Phloem.

In den folgenden Beispielen wird die Erfindung im einzelnen beschrieben. Prozentangaben beziehen sich auf das Gewicht, sofern nicht anders angegeben.

Beispiel 1

Bestimmung der Form und der Lungengängigkeit der echogenen Partikel

Die durchschnittliche Größe, die Form und die Oberflächenbeschaffenheit werden mittels rasterelektronischen Aufnahmen charakterisiert.

Dazu werden die echogenen Partikel auf ein leitfähiges Doppelklebeband aufgestreut und in einer Sputteranlage mit Gold bedampft - Schichtdicke etwa 1,8 nm, Druck etwa 1 x $10^{-2}$ mbar (Sputteranlage Typ 07 120B, Balzerz Union). Die Herstellung der REM-Bilder erfolgt mit einem Camscan-Rasterelektronenmikroskop, Typ Serie 4; Kippwinkel 15 °; Hochspannung 20 kV, Arbeitsabstand 24 mm; Sekundärelektronen, Arbeitsvakuum etwa 2 · $10^{-6}$ mbar.

Jeweils 30 mg Partikel werden in 2 ml Suspensionshilfsmittel, bestehend aus 200 mg Dextran 40 (Firma Roth, Deutschland), 10 mg Polysorbat und 16 mg NaCl in destilliertem Wasser dispergiert und anschließend mit Siebgeweben (15 µm und 3 µm Maschenweite) filtriert sowie lyophilisiert.

Die Lungengängigkeit der Partikel wird in vivo getestet. 30 mg der filtrierten Partikel werden in 1,5 ml destilliertem Wasser mit Hilfe eines Glasstabes resuspendiert. Mit einer Injektionsspritze wird diese Suspension in eine periphere Vene injiziert. Ein Ultraschallkopf eines Ultraschallgerätes (Toshiba, FSH 160a, Japan) wird an den Thorax des Versuchstieres gehalten, so daß ein typischer Querschnitt durch die rechte und linke Herzkammer erhalten wird. Sobald das Ultraschall-Kontrastmittel die rechte Herzkammer erreicht, kann man auf dem Monitor des Ultraschallgerätes erkennen, wie das durch das Kontrastmittel markierte Blut den rechten Vorhof erreicht, dann den rechten Ventrikel und anschließend das Herz über die Pulmonalarterie wieder verläßt. Nach der Lungenpassage wird der Kontrast in der linken Herzkammer durch das Kontrastmittel deutlich verstärkt.

Beispiel 2

Herstellung von Polysuccinimid-co-α,β-(hydroxy-ethyl)-D,L-aspartamid (70 : 30)

10 g (103 mmol) Polyanhydroasparaginsäure werden in etwa 40 ml N,N-Dimethylformamid (DMF) gegebenenfalls unter vorsichtigen Erwärmen gelöst. Zu dieser Lösung werden 1,83 g (30 mmol) frisch destilliertes 2-Aminoethanol zugetropft und bei Raumtemperatur über Nacht gerührt. Der Reaktionsansatz wird in Butanol gefällt und mit getrocknetem Aceton mehrfach gewaschen. Die Trocknung erfolgt im Vakuum bei erhöhter Temperatur. Das weiße, wasserlösliche Produkt entsteht zu annähernd 100 % und wird NMR-spektroskopisch auf Rückstände von DMF und Butanol geprüft. Das eingesetzte molare Verhältnis von Polyanhydroasparaginsäure zu Aminoethanol entspricht etwa der Copolymerenzusammensetzung.

Beispiel 3

Herstellung von Polysuccinimid-co-α,β-(nonyl-carbonyloxyethyl)-D,L-aspartamid (50 : 50)

6 g eines Polysuccinimid-co-α,β-(hydroxyethyl)-D,L-aspartamids (50 : 50) (= 24 mmol Hydroxyethylgruppen), das analog Beispiel 4 aus Polyanhydroasparaginsäure (MG = 14000) und 2-Aminoethanol (molares Verhältnis 2:1) hergestellt wurde werden in 100 ml trockenem DMF gelöst, mit 8 g (100 mmol) trockenem Pyridin versetzt und auf 0 °C gekühlt. Es werden langsam 9,6 g destilliertes Decansäurechlorid zugetropft.

Der Ansatz wird über Nacht gerührt und in 0,51 Ether gefällt. Das ausgefallene Produkt wird abgesaugt, mit Ether, Aceton, Wasser, Aceton und Ether gewaschen.

Man erhält etwa 8 g eines weißen, vollständig substituierten Polymers (NMR-Kontrolle), das z. B. in Dichlormethan und THF mit jeweils einer Spur DMSO oder in Methano/Dichlormethan-Gemischen löslich ist.

Beispiel 4

Herstellung von Polysuccinimid-co-α,β-(nonyl-carbonyloxyethyl)-D,L-aspartamid

Analog Beispiel 2 werden verschiedene Polysuccinimid-co-α,β-(hydroxyethyl)-D,L-aspartamide u. a. der Zusammensetzung 70 : 30, 50 : 50 und 30: 70 aus Polyanhydroasparaginsäuren unterschiedlichen Molekulargewichtes (MG = 7000; ca. 13000; 30000) hergestellt und mit Decansäurechlorid, wie in Beispiel 5 beschrieben zu den entsprechenden Polysuccinimid-co-α,β-(nonylcarbonyloxyethyl)-D,L-aspartamiden umgesetzt.

a) - Polysuccinimid-co-α,β-(nonylcarbonyloxy-ethyl)-D,L-aspartamid (70 : 30) aus Polyanhydroasparaginsäure (MG = 7000); charakterisiert durch NMR.
b) - Polysuccinimid-co-α,β-(nonylcarbonyloxy-ethyl)-D,L-aspartamid (70 : 30) aus Polyanhydroasparaginsäure (MG = 14000); charakterisiert durch NMR.
c) - Polysuccinimid-co-α,β-(nonylcarbonyloxy-ethyl)-D,L-aspartamid (70:30) aus Polyanhydroasparaginsäure (MG = 30000); charakterisiert durch NMR.

Beispiel 5

20 g von Polyanhydroasparaginsäure-co-α,β-(nonylcarbonyloxyethyl)-D,L-aspartamid 70 : 30 (hergestellt wie in Beispiel 6b) werden in 400 ml Dichlormethan gelöst und unter folgenden Bedingungen in einem Sprühtrockner (Min Spray Dryer Büchi 190, Büchi GmbH, Eislingen) zu Partikeln versprüht:

Sprühbedingungen

| | |
|---|---|
| Eingangstemperatur | 60 °C |
| Ausgangstemperatur | 40 °C |
| Skalenteile Heizung | 1,5 |
| Pumpeneinstellung | 2 |
| Aspirator | 12 |
| Sprühflow Düsenluft | 700 Nl/h |
| Druck im Filter | - 55 mbar |
| Kühlung Düse | 20 °C |
| Ausbeute | ca. 8 g |

Nach der Sprühtrocknung beträgt die Dichlormethankonzentration in den Partikeln weniger als 0,05 %. Die durchschnittliche Größe, Form und die Oberflächenbeschaffenheit wird mittels rasterelektronischen Aufnahmen charakterisiert; die Lungengängigkeit der Partikel wird wie in Beispiel 1 bestimmt.
100 mg der hergestellten Partikel werden in einer 1 %igen ®Haemaccellösung (Behring Werke AG, Marburg, Deutschland) suspendiert und durch Siebgewebe (10 μm) filtriert. Das Filtrat wird anschließend lyophilisiert und zur Bestimmung der Lungengängigkeit verwendet.
Die hergestellten Partikel haben im Rasterelektronenmikroskop eine durchschnittliche Partikelgröße von 2 μm und eine glatte Oberfläche. Ferner sind diese Partikel lungengängig.
Etwa 15 % der Partikel sind runde Hohlkugeln, 50 % der Partikel sind Hohlkugeln, die eine kugelförmige Einstülpung aufweisen, so daß eine halbkugelförmige Hohlkugel entsteht. Die Schalendicke dieser halbkugelförmigen Hohlkugeln läßt sich bei maximaler Einstülpung durch die Gleichung $r_m - r_i = 2$ d beschreiben (d = Schalendicke, $r_m$ = Radius der äußeren Halbkugel, $r_i$ = Radius der inneren konkaven Halbkugel. Wenn eine nichtideale Halbkugelform vorliegt, so ist der Radius vom relativen Mittelpunkt der eingestülpten Hohlkugel zu bestimmen.
Etwa 25 % der Partikel sind Hohlkugeln mit 2 bis 5 unterschiedlich großen Einstülpungen.

Beispiel 6

Aus Polyhydroxybuttersäure Typ 159PHB (ICI Biological Products Ltd) werden nach einer Vorschrift von J. K. Embleton, B.J. Tighe (J. Microencapsulation, 1992, Vol 9, No 1, 73-87) Partikel durch sogen. "Doppelten Emulsions Lösungsmittel-Verdampfungsprozeß" hergestellt. Die Verdampfung erfolgt bei Raumtemperatur. Die Partikel werden durch Zentrifugation und Filtration durch ein 10 μm Siebgewebe fraktioniert. Danach wird ein Partikelanteil von 35 % mit konkaven Oberflächensegmenten im Filtrat rasterelektronenmikroskopisch ermittelt.
Ebenso wird die Verdampfung bei 40°C durchgeführt und danach fraktioniert. Auf diese Weise wird zum Vergleich eine Fraktion mit einem Partikelanteil von nur 5 % mit konkaven Oberflächensegmenten rasterelektronenmikroskopisch bestimmt.

Beispiel 7 (LMFG 50/50)

Herstellung von Poly(L-Lysinmethylesterfumarsäure/L-Lysinmethylesterglutaramid)

Copolymer 50 : 50 (LMFG 50 : 50)

0,7 g Glutarsäuredichlorid und 0,84 g Fumamäuredichlorid werden in 170 ml $CH_2Cl_2$ gelöst. Diese Lösung wird bei Raumtemperatur unter kräftigem Rühren zu einer Lösung von 2,91 g L-Lysinmethylester und 3,0 g $Na_2CO_3$ in 120 ml $H_2O$ gegeben. Die schlagartig eintretende Polykondensation wird nach 15 Minuten durch Zugabe von 120 ml 1 N wäßriger HCI abgebrochen. Durch Einleiten von Wasserdampf wird dann das Methylenchlorid ausgetrieben. Das heiße wäßrige Gemisch wird filtriert und das feste Produkt mehrmals mit Wasser gewaschen.
Ausbeute: 1,3 g (60 % d. Th.) nach Vakuumtrocknung (20 Stunden).

Beispiel 8

2 g Polymer aus Beispiel 7 werden in 100 ml DMSO gelöst und bei ca. 200 °C Eingangstemperatur in einem Mini Spray Dryer Büchi 190 zu Partikeln versprüht. Die durchschnittliche Größe und Oberflächenbeschaffenheit wird mittels rasterelektronischen Aufnahmen charakterisiert (s. Tab. 1). Jeweils 50 mg Partikel werden in 2 ml Suspensionshilfsmittel (1 % ®Haemaccel in isotonem Wasser) suspendiert und anschließend mit Siebgeweben (15 μm und 3 μm Maschenweite) filtriert sowie lyophilisiert.

Beispiel 9

2 g Polyhydroxybuttersäure Typ 159 PHB (ICI Biological Products Ltd.) werden in 100 ml Furan-Dichlormethan (9 : 1) Gemisch gelöst und bei ca. 45 °C im Büchi Mini Spray Dryer zu Partikeln versprüht und analog zu Beispiel 8 weiterverarbeitet.

Beispiel 10

Eine Lösung von 2 g Polyhydroxybuttersäure Typ 159 PHB (ICI Biological Products Ltd.) in 100 ml Dichlormethan/MeOH (1 : 2) wird bei ca. 45 °C im Büchi Mini Spray Dryer zu Partikeln versprüht und analog zu Beispiel 8 weiterverarbeitet.

Beispiel 11

2 g Poly-e-caprolacton (Polysciences 19561) werden zu 2 % in Dichlormethan gelöst und bei Raumtemperatur im Büchi Mini Spray Dryer zu Partikeln versprüht und analog zu Beispiel 8 weiterverarbeitet.

Die Lungengängigkeit der Partikel aus den Beispielen 8 bis 11 wird in vivo getestet und wie in Beispiel 1 bestimmt.

Die nachfolgende Tabelle zeigt die Ergebnisse:

Tabelle 1

| Polymer nach Beispiel | Lösungsmittel | durchschnittliche Partikelgröße | Oberfläche | Lungengängigkeit |
|---|---|---|---|---|
| 8 | DMSO | 3 µm | glatt | ja |
| 9 | Furan/Dichlormethan 9 : 1 | 3 µm | glatt | ja |
| 10 | $CH_2Cl_2$/MeOH 1 : 2 | 2 µm | rauh | nein |
| 11 | $CH_2Cl_2$ | 3 µm | glatt | ja |

## Patentansprüche

### Patentansprüche für folgende Vertragsstaaten : AT, BE, CH, DE, DK, FR, GB, IT, IE, IL, LU, MC, NL, PT, SE

1. Echogene Partikel, enthaltend ein Gas und eine formgebende Substanz oder ein Substanzengemisch, die/das biologisch abbaubar und/oder ausscheidbar ist, und im Blutkreislauf die Lunge von Wirbeltieren passieren können, **dadurch gekennzeichnet, daß** (a) die Oberfläche der echogenen Partikel im wesentlichen glatt ist und die Form der echogenen Partikel keine oder 1 bis 10 konkave Oberflächensegrnente enthält oder die Oberfläche der echogenen Partikel nicht glatt ist und die Form der echogenen Partikel 1 bis 10 konkave Oberflächensegrnente enthält, und **daß** (b) die maximale Einwölbung des konkaven Oberflächensegments von 30 % bis 90 % des kleinsten Partikelquerschnitts beträgt und 10 bis 60 % der echogenen Partikel mindestens ein konkaves Oberflächensegment pro Partikel aufweisen.

2. Echogene Partikel nach Anspruch 1, **dadurch gekennzeichnet, daß** als formgebende Substanz mindesten ein ist Polymer aus der folgenden Gruppe enthalten ist: Polyester von α-, β-, γ- oder ε-Hydroxycarbonsäuren; Copolymere der Polyester untereinander oder mit Polyglykolsäure; Polyamide; Polyaminosäuren; Polyorthoester; Polykondensate die 2,3-O-Alkylidenweinsäure, 2,3-O-Alkyliden-L-threitol, Furo-[2,5]-gruppen oder Terephthalate enthalten; Poyaminodicarbonsäure-co-imide; Polyanhydride; Polymethylenmalonat; Gelatine; Albumine oder Polysaccharide oder mindestens eine formgebende Substanz aus der Gruppe Zucker; Fettsäuren; Aminosäuren; Alkohole mit 12 bis 20 Kohlenstoffatomen oder Lipide enthält.

3. Echogene Partikel nach Anspruch 1 oder 2, **dadurch gekennzeichnet daß** als formgebende Substanz mindemist stens ein Polymer aus der folgenden Gruppe enthalten ist: Poly-ε-caprolacton; Polyhydroxybuttersäure; Polymilchsäure; Polyhydroxyvaleriansäure; Polyglutaminsäure; Polyasparaginsäure; Polylysin; Polysuccinimid-co-α,β-(butyl-oxycarbonyl-propionyloxyethyl)-D,L-aspartamid; Polyanhydride aus Sebacinsäure und Bis(p-carboxyphenoxy) propan; Polylysinmethylesterfumarsäure-co-glutaramid Dextrane; Stärke; basische oder saure Derivate von Dextranen oder Stärke; Polyhydroxyethylaspartamid; Rinderserumalbumin; hydrophob modifizierte Gelatine sowie mindestens eine formgebende Substanz aus folgender Gruppe Glucose; Galaktose; Laktose; Saccharose, Fettsäuren mit 8 bis 20 C-Atomen, proteinogene Aminosäuren, hydrophobe Derivate von proteinogenen Aminosäuren, Phospholipide oder Mischungen derselben enthält.

4. Echogene Partikel nach einem oder mehreren der Ansprüche 1 bis 3, **dadurch gekennzeichnet, daß** als formmist gebende Subtanz mindestens ein Polymer aus folgender Gruppe enthalten ist: Polyamide; Polykondensate Polyhydroxybuttersäure oder Polyaminodicarbonsäure-co-imide enthalten sind.

5. Echogene Partikel nach einem oder mehreren der Ansprüche 1 bis. 4, **dadurch gekennzeichnet, daß** die maximale Einwölbung des konkaven Oberflächensegments 50 % bis 90 % des kleinsten Partikelquerschnitts beträgt.

6. Echogene Partikel nach einem oder mehreren der Ansprüche 1 bis 5, **dadurch gekennzeichnet daß** sie als Gas Luft, Stickstoff, ein Edelgas, Wasserstoff, Kohlendioxid, Sauerstoff oder Gemische derselben enthalten.

7. Echogene Partikel nach einem oder mehreren der Ansprüche 1 bis 6, **dadurch gekennzeichnet, daß** die Partikel monolitisch sind oder als Hohlkörper vorliegen.

8. Echogene Partikel nach Anspruch 7, **dadurch gekennzeichnet, daß** der Hohlkörper 2 bis 10 konkave Oberflächensegmente aufweist.

9. Echogene Partikel nach Anspruch 7, **dadurch gekennzeichnet, daß** 30 bis 40 % der echogenen Partikel mindestens ein konkaves Oberflächensegment pro Partikel aufweisen.

10. Echogene Partikel nach einem oder mehreren der Ansprüche 1 bis 9, **dadurch gekennzeichnet, daß** die Oberfläche 0 bis 4, insbesondere 1 bis 3, Störungen pro $\mu m^2$ aufweist.

11. Echogene Partikel nach Anspruch 10, **dadurch gekennzeichnet daß** mindestens 10 %, insbesondere 20 bis 60 %, der echogenen Partikel 0 bis 4, insbesondere 1 bis 3, Störungen pro $\mu m^2$ aufweisen.

12. Verfahren zur Herstellung von echogenen Partikeln nach einem oder mehreren der Ansprüche 1 bis 11, **dadurch gekennzeichnet, daß** die formgebende Substanz oder das Substanzengemisch in einem geeigneten Lösungsmittel oder Lösungsmittelgemisch gelöst wird und die Lösung anschließend einer Sprühtrocknung unterworfen wird.

13. Diagnostikum oder Therapeutikum, enthaltend echogene Partikel nach einem oder mehreren der Ansprüche 1 bis 11 oder hergestellt nach dem Verfahren gemäß Anspruch 12, neben pharmazeutisch geeigneten und physiologisch verträglichen Trägerstoffen und gegebenenfalls weiteren Zusatz- und/oder Hilfsstoffen.

14. Verfahren zur Herstellung eines Diagnostikums oder Therapeutikums nach Anspruch 13, **dadurch gekennzeichnet, daß** man echogene Partikel nach einem oder mehreren der Ansprüche 1 bis 11 oder hergestellt nach Anspruch 12 mit einem pharmazeutisch geeigneten und physiologisch verträglichen Träger- und gegebenenfalls weiteren Zusatz- und/oder Hilfsstoffen in eine geeignete diagnostische oder therapeutische Darreichungsform bringt.

15. Verfahren nach Anspruch 14, **dadurch gekennzeichnet, daß** man die echogenen Partikel durch ein Siebgewebe mit einer Maschenweite von 1 $\mu m$ bis 50 $\mu m$, insbesondere 3 $\mu m$ bis 15 $\mu m$ filtriert.

16. Verwendung der echogenen Partikel nach einem oder mehreren der Ansprüche 1 bis 11 oder hergestellt nach dem Verfahren nach Anspruch 12 zur Herstellung von Diagnostika zur Sichtbarmachung des Blutkreislaufs oder von Organen, die mit Blut versorgt werden.

17. Verwendung der echogenen Partikel nach einem oder mehreren der Ansprüche 1 bis 11 zur Herstellung eines Diagnostikums zur Kontrastierung von Hohlräumen in Menschen, Tieren oder Pflanzen.

**Patentansprüche für folgende Vertragsstaaten : ES, GR**

1. Verfahren zur Herstellung von echogenen Partikel, enthaltend ein Gas und eine formgebende Substanz oder ein Substanzengemisch, die/das biologisch abbaubar und/oder ausscheidbar ist, und im Blutkreislauf die Lunge von Wirbeltieren passieren können, wobei (a) die Oberfläche des echogenen Partikels keine oder 1 bis 10 konkave Oberflächensegmente enthält oder die Oberfläche des echogenen Partikels nicht glatt ist und die Form des echogenen Partikels 1 bis 10 konkave Oberflächensegmente enthält, und wobei (b) die maximale Einwölbung des konkaven Oberflächensegments von 30 % bis 90 % des kleinsten Partikelquerschnitts beträgt und 10 bis 60 % der echogenen Partikel mindestens ein konkaves Oberflächensement pro Partikel aufweisen, **dadurch gekennzeichnet, daß** man eine formgebende Substanz oder ein Substanzengemisch in einem geeigneten Lösungsmittel oder Lösungsmittelgemisch löst und sprühtrocknet.

2. Verfahren nach Anspruch 1, **dadurch gekennzeichnet, daß** als formgebende Substanz mindestens ein Polymer aus der Gruppe: Polyester von $\alpha$-, $\beta$-, $\gamma$- oder $\varepsilon$-Hydroxycarbonsäuren; Copolymere der Polyester untereinander oder mit Polyglykolsäure; Polyamide; Polyaminosäuren; Polyorthoester; Polykondensate, die 2,3-O-Alkylidenweinsäure, 2,3-O-Alkyliden-L-threitol, Furo-[2,5]-gruppen oder Terephthalate enthalten; Polyaminodicarbonsäure-co-imide; Polyanhydride; Polymethylenmalonat; Gelatine; Albumine oder Polysaccharide oder mindestens eine formgebende Substanz aus der Gruppe Zucker; Fettsäuren; Aminosäuren; Alkohole mit 12 bis 20 Kohlenstoffatomen oder Lipide verwendet wird.

**3.** Verfahren nach Anspruch 1 oder 2, **dadurch gekennzeichnet, daß** als formgebende Substanz mindestens ein Polymer aus der folgenden Gruppe: Poly-ε-caprolacton; Polyhydroxybuttersäure; Polymilchsäure; Polyhydroxyvaleriansäure; Polyglutaminsäure; Polyasparaginsäure; Polylysin; Polysuccinimid-co-α,β-(butyl-oxycarbonylpropionyloxyethyl)-D,L-aspartamid; Polyanhydride aus Sebacinsäure und Bis(p-carboxyphenoxy)propan; Polylysinmethylesterfumarsäure-co-glutaramid Dextrane; Stärke; basische oder saure Derivate von Dextranen oder Stärke; Polyhydroxyethylaspartamid; Rinderserumalbumin; hydrophob modifizierte Gelatine sowie mindestens eine formgebende Substanz aus folgender Gruppe Glucose; Galaktose; Laktose; Saccharose, Fettsäuren mit 8 bis 20 C-Atomen. proteinogene Aminosäuren, hydrophobe Derivate von proteinogenen Aminosäuren, Phospholipide oder Mischungen derselben verwendet wird.

**4.** Verfahren nach einem oder mehreren der Ansprüche 1 bis 3, **dadurch gekennzeichnet, daß** als formgebende Subtanz mindestens ein Polymer aus folgender Gruppe: Polyamide; Polykondensate; Polyhydroxybuttersäure oder Polyaminodicarbonsäure-co-imide verwendet wird.

**5.** Verfahren nach einem oder mehreren der Ansprüche 1 bis 4, **dadurch gekennzeichnet, daß** man Partikel erhält, deren maximale Einwölbung des konkaven Oberflächensegments von 50 % bis 90 % des kleinsten Partikelquerschnitts beträgt.

**6.** Verfahren nach einem oder mehreren der Ansprüche 1 bis 5, **dadurch gekennzeichnet, daß** die Sprühtrocknung in Luft, Stickstoff, Edelgas, Wasserstoff, Kohlendioxid, Sauerstoff oder Gemischen derselben durchgeführt wird.

**7.** Verfahren nach einem oder mehreren der Ansprüche 1 bis 6, **dadurch gekennzeichnet, daß** man Partikel erhält, die monolitisch sind oder als Hohlkörper vorliegen.

**8.** Verfahren nach Anspruch 7, **dadurch gekennzeichnet, daß** man Hohlkörper mit 2 bis 10 konkaven Oberflächensegmenten erhält.

**9.** Verfahren nach Anspruch 8, **dadurch gekennzeichnet, daß** 30 bis 40 % der enthaltenen echogenen Partikel mindestens ein konkaves Oberflächensegment pro Partikel aufweisen.

**10.** Verfahren nach einem oder mehreren der Ansprüche 1 bis 9, **dadurch gekennzeichnet, daß** man Partikel erhält, deren Oberfläche 0 bis 4, insbesondere 1 bis 3 Störungen pro $\mu m^2$ aufweisen.

**11.** Verfahren nach Anspruch 10, **dadurch gekennzeichnet, daß** mindestens 10 %, insbesondere 20 bis 60 %, der erhaltenen echogenen Partikel 0 bis 4, insbesondere 1 bis 3 Störungen pro $\mu m^2$ aufweisen.

**12.** Verfahren zur Herstellung eines Diagnostikums oder Therapeutikums, **dadurch gekennzeichnet, daß** man echogene Partikel nach einem oder mehreren der Ansprüche 1 bis 11 mit einem pharmazeutisch geeigneten und physiologisch verträglichen Träger- und gegebenenfalls weiteren Zusatz- und/oder Hilfsstoffen in eine geeignete diagnostische oder therapeutische Darreichungsform bringt.

**13.** Verfahren nach Anspruch 12, **dadurch gekennzeichnet, daß** man die echogenen Partikel durch ein Siebgewebe mit einer Maschenweite von 1 µm bis 50 µm, insbesondere 3 µm bis 15 µm filtriert.

**14.** Verwendung der echogenen Partikel nach einem oder mehreren der Ansprüche 1 bis 11 oder hergestellt nach dem Verfahren nach Anspruch 12 zur Herstellung von Diagnostika zur Sichtbarmachung des Blutkreislaufs oder von Organen, die mit Blut versorgt werden.

**15.** Verwendung der echogenen Partikel nach einem oder mehreren der Ansprüche 1 bis 11 zur Herstellung eines Diagnostikums zur Kontrastierung von Hohlräumen in Menschen, Tieren oder Pflanzen.

## Claims

### Claims for the following Contracting States : AT, BE, CH, DE, DK, FR, GB, IE, IT, LI, LU, MC, NL, PT, SE

1. Echogenic particles comprising a gas and a shaping substance or a substance mixture, which is biologically degradable and/or excretable, and can pass through the lungs of vertebrates in the blood circulation, wherein (a) the surface of the echogenic particles is essentially smooth and the shape of the echogenic particles comprises no or 1 to 10 concave surface segments, or the surface of the echogenic particles is not smooth and the shape of the echogenic particles comprises 1 to 10 concave surface segments, and wherein (b) the maximum invagination of the concave surface segment is from 30% to 90% of the smallest partcle cross-section and 10 to 60% of the echogenic partcles have at least one concave surface segment per particle.

2. Echogenic particles as claimed in claim 1, which comprise, as the shaping substance, at least one polymer from the following group: polyesters of α-, β-,γ- or ε-hydroxycarboxylic acids; copolymers of the polyesters with one another or with polyglycolic acids; copolymers of the polyesters with one another or with polyglycolic acid; polyamides; polyamino acids; polyorthoesters; polycondensates which comprise 2,3-O-alkylidenetartaric acid, 2,3-O-alkylidene-L-threitol, furo-[2,5] groups or terephthalates; polyaminodicarboxylic acid co-imides; polyanhydrides; polymethylene malonate; gelatin; albumins and polysaccharides, or comprise at least one shaping substance from the group consisting of sugars; fatty acids; amino acids; alcohols having 12 to 20 carbon atoms and lipids.

3. Echogenic particles as claimed in claim 1 or 2, which comprise , as the shaping substance, at least one polymer from the following group: poly-ε-caprolactone; polyhydroxybutyric acid; polylactic acid; polyhydroxyvaleric acid; polyglutamic acid; polyaspartic acid, polylsine; polysuccinimide-co-α,β-(butyloxycarbonyl-propionyloxyethyl)-D-L-aspartamide; polyanhydrides of sebacic acid and bis(p-carboxyphenoxy)propane; polylsine methyl ester-fumaric acid co-glutaramide; dextrans; starch; basic or acid derivatives of dextrans or starch; polyhydroxyethylaspartamide; bovine serum albumin; and hydrophobically modified gelatin, and at least one shaping substance from the following group: glucose; galactose; lactose; sucrose, fatty acids having 8 to 20 carbon atoms, proteinogenic amino acids, hydrophobic derivatives of proteinogenic amino acids and phospholipids, or mixtures thereof.

4. Echogenic particles as claimed in one or more of claims 1 to 3, which comprise, as the shaping substance, at least one polymer from the following group: polyamides; polycondensates; polyhydroxybutyric acid and polyaminodicarboxylic acid co-imides.

5. Echogenic particles as claimed in one or more of claims 1 to 4, wherein the maximum invagination of the concave surface segment 50% to 90% of the smallest particle cross section.

6. Echogenic particles as claimed in one or more of claims 1 to 5, which comprise, as the gas, air, nitrogen, a noble gas, hydrogen, carbon dioxide, oxygen or mixtures thereof.

7. Echogenic particles as claimed in one or more of claims 1 to 6, wherein the particles are monolythic or in the form of a hollow body.

8. Echogenic particles as claimed in claim 7, wherein the hollow body has 2 to 10 concave surface segments.

9. Echogenic particles as claimed in claim 7, wherein 30 to 40% of the echogenic particles have at least one concave surface segment per particle.

10. Echogenic particles as claimed in one or more of claims 1 to 9, wherein the surface has 0 to 4, in particular 1 to 3, disturbances per $\mu m^2$.

11. Echogenic particles as claimed in claim 10, wherein at least 10%, in particular 20 to 60%, of the echogenic particles have 0 to 4, in paticular 1 to 3, disturbances per $\mu m^2$.

12. A process for the production of echogenic particles as claimed in one or more of claims 1 to 11, which comprises dissolving the shaping substance or the substance mixture in a suitable solvent or solvent mixture and then subjecting the solution to spray drying.

**13.** A diagnostic agent or therapeutic agent comprising echogenic particles as claimed in one or more of claims 1 to 11 or produced by the process as claimed in claim 12, in addition to pharmaceutically suitable and physiologically tolerated excipients and if appropriate other additives and/or auxiliaries.

**14.** A process for the preparation of a diagnostic agent or therapeutic agent as claimed in claim 13, which comprises bringing echogenic particles as claimed in one or more of claims 1 to 11 or produced as claimed in claim 12 into a suitable diagnostic or therapeutic presentation form with a pharmaceutically suitable and physiologically tolerated excipient and if appropriate other additives and/or auxiliaries.

**15.** The process as claimed in claim 14, wherein the echogenic particles are filtered through a sieve fabric having a mesh width of 1μm to 50μm, in particular 3μm to 15μm.

**16.** The use of the echogenic particles as claimed in one or more of claims 1 to 11 or produced by the process as claimed in claim 12 for the preparation of diagnostic agents for visualization of the blood circulation or of organs supplied with blood.

**17.** The use of the echogenic particles as claimed in one or more of claims 1 to 11 for the preparation of a diagnostic agent for contrasting hollow cavities in humans, animals or plants.

**Claims for the following Contracting States : ES, GR**

**1.** A process for the production of echogenic particles which comprise a gas and a shaping substance or substance mixture, which is biologically degradable and/or excretable, and can pass through the lungs of vertebrates in the blood circulation, wherein (a) the surface of the echogenic particle comprises no or 1 to 10 concave surface segments, or the surface of the echogenic particle is not smooth and the shape of the echogenic particle comprises 1 to 10 concave surface segments, and wherein (b) the maximum invagination of the concave surface segment is from 30% to 90% of the smallest particle cross-section and 10 to 60% of the echogenic particles have at least one concave surface segment per particle, which process comprises dissolving a shaping substance or a substance mixture in a suitable solvent or solvent mixture and spray drying the solution.

**2.** The process as claimed in claim 1, wherein the shaping substance used is at least one polymer from the group consisting of: polyesters of α-, β-,γ- or ε-hydroxycarboxylic acids; copolymers of the polyesters with one another or with polyglycolic acid; polyamides; polyamino acids; polyorthoesters; polycondensates which comprise 2,3-O-alkylidenetartaric acid, 2,3-O-alkylidene-L-threitol, furo-[2,5] groups or terephthalates; polyaminodicarboxylic acid co-imides; polyanhydrides; polymethylene malonate; gelatin; albumins and polysaccharides, or comprises at least one shaping substance from the group consisting of sugars; fatty acids; amino acids; alcohols having 12 to 20 carbon atoms and lipids.

**3.** The process as claimed in claim 1 or 2, wherein the shaping substance used is at least one polymer from the following group: poly-ε-caprolactone; polyhydroxybutyric acid; polylactic acid; polyhydroxyvaleric acid; polyglutamic acid; polyaspartic acid; polylsine; polysuccinimide-co-α,β-(butyloxycarbonyl-propionyloxyethyl)-D-L-aspartamide; polyanhydrides of sebacic acid and bis(p-carboxyphenoxy)propane; polylsine methyl ester-fumaric acid co-glutaramide; dextrans; starch; basic or acid derivatives of dextrans or starch; polyhydroxyethylaspartamide; bovine serum albumin; and hydrophobically modified gelatin, and at least one shaping substance from the following group: glucose; galactose; lactose; sucrose, fatty acids having 8 to 20 carbon atoms, proteinogenic amino acids, hydrophobic derivatives of proteinogenic amino acids and phospholipids, or mixtures thereof.

**4.** The process as claimed in one or more claims 1 to 3, wherein the shaping substance used is at least one polymer from the following group: polyamides; polycondensates; polyhydroxybutyric acid and polyaminodicarboxylic acid co-imides.

**5.** The process as claimed in one or more claims 1 to 4, wherein particles whose maximum invagination of the concave surface segment is from 50% to 90% of the smallest particle cross-section are obtained.

**6.** The process as claimed in one or more claims 1 to 5, wherein the spray drying is carried out in air, nitrogen, noble gas, hydrogen, carbon dioxide, oxygen or mixtures thereof.

7. The process as claimed in one or more claims 1 to 6, wherein particles which are monolithic or in the form of a hollow body are obtained.

8. The process as claimed in claim 7, wherein hollow bodies having 2 to 10 concave segments are obtained.

9. The process as claimed in claim 8, wherein 30 to 40% of the echogenic particles have at least one concave surface segment per particle.

10. The process as claimed in one or more of claims 1 to 9, wherein particles whose surface has 0 to 4, in particular 1 to 3, disturbances per $\mu m^2$ are obtained.

11. The process as claimed in claim 10, wherein at least 10% in particular 20 to 60%, of the echogenic particles obtained have 0 to 4, in particular 1 to 3, disturbances per $\mu m^2$.

12. A process for the preparation of a diagnostic agent or therapeutic agent, which comprises bringing echogenic particles as claimed in one or more of claims 1 to 11 into a suitable diagnostic or therapeutic presentation form with a pharmaceutically suitable and physiologically tolerated excipient and if appropriate other additives and/or auxiliaries.

13. The process as claimed in claim 12, wherein the echogenic particles are filtered through a sieve fabric having a mesh width of 1μm to 50μm, in particular 3μm to 15μm.

14. The use of the echogenic particles as claimed in one or more claims 1 to 11 or produced by the process as in claim 12 for the preparation of diagnostic agents for visualization of the blood circulation or of organs supplied with blood.

15. The use of the echogenic particles as claimed in one or more claims 1 to 11 for the preparation of a diagnostic agent for contrasting hollow cavities in humans, animals or plants.

## Revendications

**Revendications pour les Etats contractants suivants : AT, BE, CH, DE, DK, FR, GB, IE, IT, LI, LU, MC, NL, PT, SE**

1. Particules échographiques contenant un gaz et une substance ou un mélange de substances leur donnant sa forme, qui est biologiquement dégradable et/ou peut être excrété(e), apte à traverser le poumon de vertébrés dans la circulation sanguine, caratérisées en ce que (a) la surface des particules échographiques est pratiquement lissée et la forme des particules échographiques ne comprend pas de segment superficiel ou comprend 1 à 10 segments superficiels concaves, ou la surface des particules échographiques n'est pas lissée et la forme des particules échographiques comprend 1 à 10 segments superficiels concaves, et en ce que (b) le bombement maximum du segment superficiel concave représentant 30 à 90 % de la plus petite section transversale de la particule et 10 à 60 % des particules échographiques présentant au moins un segment superficiel concave par particule.

2. Particules échographiques selon la revendication 1, **caractérisées en ce qu'**elles contiennent comme substance leur donnant ses formes au moins un polymère choisi dans le groupe suivant: polyesters d'acides α-, β-, γ- ou ε-hydroxycarboxyliques; copolymères des polyesters entre eux ou avec le poly(acide glycolique); polyamides; polyaminoacides; polyorthoesters; produits de polycondensation qui contiennent un acid 2,3-O-alkylidènetartrique, un 2,3-O-alkylidène-L-thréitol, des groupes furo-(2,5) ou des téréphtalates; poly(acide aminodicarboxylique-co-imide)s; polyanhydrides; le poly(malonate de méthylène); gélatines; albumines ou polysaccharides, ou au moins une substance donnant les formes, choisie parmi des sucres des acides gras, des aminoacides, des alcools ayant de 12 à 20 atomes de carbone ou des lipides.

3. Particules échographiques selon la revendication 1 ou 2, **caractérisées en ce qu'**elles contiennent en tant que substance leur donnant ses formes, au moins un polymère choisi dans le groupe suivant: la poly-ε-caprolactone, le poly(acide hydroxybutyrique), le poly(acide lactique), le poly(acide hydroxyvalérique), le poly(acide glutamique), le poly (acide aspartique), la polylsine, le poly[succinimide-co-α,β-(butyloxycarbonylpropionyloxyéthyl)-D,L-aspartamide], des polyanhydrides d'acide sébacique et de bis(p-carboxyphénoxy)propane, le poly(ester méthylique de

lysine-acide fumarique-co-glutaramide), des dextrans, des amidons, des dérivés acides et basiques de dextrans ou d'amidons, le polyhydroxyéthylaspartamide, l'albumine de sérum bovin, des gélatines à modification hydrophobe ainsi qu'au moins une substance donnant les formes, choisie dans le groupe suivant: glucose, galactose, lactose, saccharose, acides gras ayant de 8 à 20 atomes de carbone, aminoacides protéinogènes, dérivés hydrophobes d'aminoacides protéinogènes, phospholipides ou mélanges de ceux-ci.

**4.** Particules échographiques selon une ou plusieurs des revendications 1 à 3, **caractérisées en ce qu'**elles contiennent, comme substance leur donnant ses formes, au moins un polymère choisi dans le groupe suivant: polyamides, produits de polycondensation, poly(acide hydroxybutyrique) ou poly(acide aminodicarboxylique-co-imide) s.

**5.** Particules échographiques selon une ou plusieurs des revendications 1 à 4, **caractérisées en ce que** le bombement maximum du segment superficiel concave représente de 50 à 90 % de la plus petite section transversale de la particule.

**6.** Particules échographiques selon une ou plusieurs des revendications 1 à 5, **caractérisées en ce qu'**elles contiennent en tant que gaz de l'air, de l'azote, un gaz rare, de l'hydrogène, du dioxyde de carbone ou des mélanges de ceux-ci.

**7.** Particules échographiques selon une ou plusieurs des revendications 1 à 6, **caractérisées en ce que** les particules sont monolithiques ou se présentent sous forme d'un corps creux.

**8.** Particules échographiques selon la revendication 7, **caractérisées en ce que** le corps creux comporte de 2 à 10 segments superficiels concaves.

**9.** Particules échographiques selon la revendication 7, **caractérisées en ce que** 30 à 40 % des particules échographiques comportent au moins un segment superficiel concave par particule.

**10.** Particules échographiques selon une ou plusieurs des revendications 1 à 9, **caractérisées en ce que** la surface présente 0 à 4, en particulier 1 à 3 accidents par $\mu m^2$.

**11.** Particules échographiques selon la revendication 10, **caractérisées en ce qu'**au moins 10 %, en particulier 20 à 60 % des particules échographiques présentent 0 à 4, en particulier 1 à 3 accidents par $\mu m^2$.

**12.** Procédé pour la fabrication de particules échographiques selon une ou plusieurs des revendications 1 à 11, **caractérisé en ce que** l'on dissout la substance ou le mélange de substances donnant la forme dans un solvant ou mélange de solvants approprié, et on soumet ensuite la solution à un séchage par atomisation.

**13.** Agent de diagnostic ou agent thérapeutique contenant des particules échographiques selon une ou plusieurs des revendications 1 à 11 ou préparé conformément au procédé selon la revendication 12, en plus de véhicules et éventuellement d'autres additifs et/ou adjuvants pharmaceutiquement appropriés et physiologiquement acceptables.

**14.** Procédé pour la préparation d'un agent de diagnostic ou d'un agent thérapeutique selon la revendication 13, **caractérisé en ce que** l'on met sous une forme d'administration diagnostique ou thérapeutique appropriée des particules échographiques selon une ou plusieurs des revendications 1 à 11 ou fabriquées selon la revendication 12, avec un véhicule et éventuellement d'autres adjuvants et/ou additifs pharmaceutiquement appropriés et physiologiquement acceptables.

**15.** Procédé selon la revendication 14, **caractérisé en ce que** l'on tamise les particules échographiques à travers une toile filtrante ayant une ouverture de mailles de 1 à 50 µm, en particulier de 3 à 15 µm.

**16.** Utilisation des particules échographiques selon une ou plusieurs des revendications 1 à 11 ou fabriquées conformément au procédé selon la revendication 12, pour la préparation d'agents de diagnostic destinés à la visualisation de la circulation sanguine ou d'organes qui sont alimentés par le sang.

**17.** Utilisation des particules échographiques selon une ou plusieurs des revendications 1 à 11, pour la préparation d'un agent de diagnostic pour faire apparaître en contraste des cavités chez l'homme, les animaux ou les plantes.

**Revendications pour les Etats contractants suivants : ES, GR**

1. Procédé pour la préparation de particules échographiques contenant un gaz et une substance ou un mélange de substances leur donnant ses formes, qui est biologiquement dégradable et/ou peut être excrété(e), et pouvant traverser le poumon de vertébrés dans la circulation sanguine, (a) la surface de la particule échographigue ne comprenant pas de segment superficiels ou comprenant 1 à 10 segments superficiels concaves ou, la surface de la particule échographique n'étant pas lisse et la forme de la particule échographique comprenant 1 à 10 segments superficiels concaves, et (b) le bombement maximum du segment superficiel concave représentant 30 à 90 % de la plus petite section transversale de la particule, et 10 à 60 % des particules échographiques comportant au moins un segment superficiel concave par particule, **caractérisé en ce que** l'on dissout dans un solvant ou mélange de solvants approprié une substance ou un mélange de substances donnant la forme, et on sèche le mélange par atomisation.

2. Procédé selon la revendication 1, **caractérisé en ce que** l'on utilise comme substance donnant la forme, au moins un polymère choisi parmi: polyesters d'acides $\alpha$-, $\beta$-, $\gamma$- ou $\varepsilon$-hydroxycarboxyliques; copolymères des polyesters entre eux ou avec le poly(acide glycolique); polyamides; polyaminoacides; polyorthoesters; produits de polycondensation qui contiennent un acid 2,3-O-alkylidène tartrique, un 2,3-O-alkylidène-L-thréitol, des groupes furo-(2,5) ou des téréphtalates; poly(acide aminodicarboxylique-co-imide)s; polyanhydrides; le poly(malonate de méthylène); gélatines; albumines ou polysaccharides, ou au moins une substance donnant la forme, choisie parmi des sucres, des acides gras, des aminoacides, des alcools ayant de 12 à 20 atomes de carbone ou des lipides.

3. Procédé selon la revendication 1 ou 2, **caractérisé en ce que** l'on utilise comme substance donnant la forme au moins un polymère choisi dans le groupe suivant: la poly-$\varepsilon$-caprolactone, le poly(acide hydroxybutyrique), le poly (acide lactique), le poly(acide hydroxyvalérique), le poly(acide glutamique), le poly (acide aspartique), la polylsine, le poly[succinimide-co-$\alpha$,$\beta$-(butyloxycarbonylpropionyloxyéthyl)-D,L-aspartamide] des polyanhydrides d'acide sébacique et de bis(p-carboxyphénoxy)propane, le poly(ester méthylique de lysine-acide fumarique-co-glutaramide), des dextrans, des amidons, des dérivés acides et basiques de dextrans ou d'amidons, le polyhydroxyéthylaspartamide, l'albumine de sérum bovin, des gélatines à modification hydrophobe ainsi qu'au moins une substance donnant la forme, choisie dans le groupe suivant: glucose, galactose, lactose, saccharose, acides gras ayant de 8 à 20 atomes de carbone, aminoacides protéinogènes, dérivés hydrophobes d'aminoacides protéinogènes, phospholipides ou mélanges de ceux-ci.

4. Procédé selon une ou plusieurs des revendications 1 à 3, **caractérisé en ce que** l'on utilise comme substance donnant la forme au moins un polymère choisi dans le groupe suivant: polyamides, produits de polycondensation, poly(acide hydroxybutyrique) ou poly(acide amino-dicarboxylique-co-imide)s.

5. Procédé selon une ou plusieurs des revendications 1 à 4, **caractérisé en ce que** l'on obtient les particules dont le bombement maximum du segment superficiel concave représente 50 à 90 % de la plus petite section transversale de la particule.

6. Procédé selon une ou plusieurs des revendications 1 à 4, **caractérisé en ce que** l'on effectue le séchage par atomisation dans de l'air, de l'azote, un gaz rare, de l'hydrogène, du dioxyde de carbone, de l'oxygène ou des mélanges de ceux-ci.

7. Procédé selon une ou plusieurs des revendications 1 à 6, **caractérisé en ce que** l'on obtient des particules monolitiques ou se présentent sous forme de corps creux.

8. Procédé selon la revendication 7, **caractérisé en ce que** l'on obtient des corps creux comportant 2 à 10 segments superficiels concaves.

9. Procédé selon la revendication 8, **caractérisé en ce que** 30 à 40 % des particules échographiques obtenues comportent au moins un segment superficiel concave par particule.

10. Procédé selon une ou plusieurs des revendications 1 à 9, **caractérisé en ce que** l'on obtient des particules dont la surface présente 0 à 4, en particulier 1 à 3 accidents par $\mu m^2$.

11. Procédé selon la revendication 10, **caractérisé en ce qu'**au moins 10 %, en particulier 20 à 60 % des particules échographiques obtenues comportent de 0 à 4, en particulier 1 à 3 accidents par $\mu m^2$.

**12.** Procédé pour la préparation d'un agent de diagnostic ou d'un agent thérapeutique, **caractérisé en ce que** l'on met sous une forme d'administration diagnostic ou thérapeutique appropriée des particules échographiques selon une ou plusieurs des revendications 1 à 11, avec un véhicule et éventuellement d'autres additifs et/ou adjuvants pharmaceutiquement et physiologiquement acceptables.

**13.** Procédé selon la revendication 12, **caractérisé en ce que** l'on tamise les particules échographiques à travers une toile filtrante ayant une ouverture de mailles de 1 à 50 µm, en particulier de 3 à 15 µm.

**14.** Utilisation des particules échographiques selon une ou plusieurs des revendications 1 à 11 ou fabriquées conformément au procédé selon la revendication 12, pour la préparation d'agents de diagnostic destinés à la visualisation de la circulation sanguine ou d'organes qui sont alimentés par le sang.

**15.** Utilisation des particules échographiques selon une ou plusieurs des revendications 1 à 11, pour la préparation d'un agent de diagnostic pour faire apparaître en contraste des cavités chez l'homme, les animaux ou les plantes.